(19) Europäisches Patentamt
European Patent Office
Office européen des brevets

(11) **EP 4 113 374 A1**

(12) **EUROPEAN PATENT APPLICATION**

(43) Date of publication:
04.01.2023  Bulletin 2023/01

(21) Application number: 21194284.2

(22) Date of filing: 01.09.2021

(51) International Patent Classification (IPC):
*G06K 9/62* (2022.01)      *G06N 3/04* (2006.01)
*G06V 10/82* (2022.01)      *G06V 40/20* (2022.01)
*A61B 5/11* (2006.01)      *A61B 5/00* (2006.01)

(52) Cooperative Patent Classification (CPC):
**G06V 40/25; A61B 5/112; A61B 5/7264;
G06K 9/6272; G06K 9/6292; G06N 3/0454;
G06V 10/82**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  02.07.2021  KR 20210087202

(71) Applicant: **Industry-Academic Cooperation
Foundation,
Dankook University
Yongin-si Gyeonggi-do 16890 (KR)**

(72) Inventor: **CHOI, Sangi-Il
08328 Seoul (KR)**

(74) Representative: **Keilitz Haines & Partner
Patentanwälte PartGmbB
Nigerstraße 4
81675 München (DE)**

(54)    **MULTI-MODAL FEW-SHOT LEARNING DEVICE FOR USER IDENTIFICATION USING WALKING
PATTERN BASED ON DEEP LEARNING ENSEMBLE**

(57)    Disclosed is multi-modal few-shot learning device for user identification using a walking pattern based on deep learning ensemble, and the device includes: a walking data collector configured to collect walking data of a user from a smart insole including any one or more of a pressure sensor, an acceleration sensor, and a gyro sensor; a preprocessor configured to convert a series of time series walking data obtained from each of the sensors included in the smart insole into a unit format data set; and an ensemble learner configured to apply an ensemble learning model that provides one final prediction by training CNN series learning and RNN series learning respectively and independently based on the unit-format data set generated by the preprocessor.

FIG.1

**Description**

TECHNICAL FIELD

The present disclosure relates to a multi-modal few-shot learning device for user identification using a walking pattern based on deep learning ensemble, and more particularly, a multi-modal few-shot learning device for user identification using a walking pattern based on deep learning ensemble, the device which is capable of collecting a user's walking data from a smart insole and identifying the user based on an ensemble learning model.

BACKGROUND

Walking is one of the typical behaviors of human beings, and analysis of a walking pattern contains a lot of information on a person's physical activities. Since the same type of normal walking exhibits different features depending on an individual, a walking pattern may also be used for purpose of biometrics such as face recognition and fingerprint recognition.

In addition, even in the same walking motion, there are differences in pattern between walking on flat ground and walking on a hill, and these variations make it difficult to extract features so as to classify a walking type which is a "step." Thus, a walking data feature extracting technology for identifying an individual using walking data in a situation where there is variation having being developed.

Meanwhile, a walking type classifying system is composed of a sensor module for acquiring sensor data and an application module for calculating a classification result based on the acquired data. Sensors used for walking gait classification include a video sensor, an electromyoraphic (EMG) sensor, a plantar pressure sensor, an acceleration sensor, a gyro sensor, and the like.

In addition, the recent development of wearable sensor technologies has led to weight reduction and simplification of equipment which is used to measure walking data. That is, various wearable devices such as smart watches, sports bands, and smart insoles have been being developed as sensor modules are miniaturized and low-power sensor technologies are developed. Since the use of wearable sensors has fewer environmental restrictions in collecting data, the data may be collected relatively easily in daily life, and, since such data has a small capacity compared to video data such as optical flow or heat map, there is an advantage in that data storage and processing are less burdensome.

Meanwhile, techniques for collecting walking gait information using a smart insole provided with various types of sensors, performing neural network analysis for each sensor type, and classifying a walking type using the analyzed information have been proposed. However, there is a problem that such techniques derive a result including an error value rather than a desired result value due to meaningless walking data according to open set gait recognition data. Such a walking walking pattern classification technique of the related art has a problem in that there is a limit in terms of accuracy. In addition, there is a demand for development of a new device capable of increasing the accuracy of walking pattern classification.

[Prior art literature]

[Patent Literature]

Korea Patent No. 10-2175191 (October 30, 2020)
Korea Number No. 10-2061810 (December 26, 2019)
Korea Number No. 10-2194313 (December 16, 2020)

SUMMARY

Anaspect of the present disclosure provides a multi-modal few-shot learning device for user identification using a walking pattern based on deep learning ensemble, the device which is capable of collecting walking data of a user from a smart insole, recognizing and excluding meaningless walking information through a neural network trained with an ensemble learning model and extracting only feature data that enables identification of the user.

According to an aspect of the present disclosure, there is provided a multi-modal few-shot learning device for user identification using a walking pattern based on deep learning ensemble, and the device includes: a walking data collector configured to collect walking data of a user from a smart insole including any one or more of a pressure sensor, an acceleration sensor, and a gyro sensor; a preprocessor configured to convert a series of time series walking data obtained from each of the sensors included in the smart insole into a unit format data set; and an ensemble learner configured to apply an ensemble learning model that provides one final prediction by training CNN series learning and RNN series learning respectively and independently based on the unit-format data set generated by the preprocessor.

According to embodiments of the present disclosure, the walking data collector may include any one or more of n pressure sensors, the acceleration sensor, and the gyro sensor included in the smart insole, and collects the walking data of the user measured from the sensors.

According to embodiments of the present disclosure, the n pressure sensors each may measure a measurement level of foot pressure of both feet of the user is as 0, 1, or 2.

According to embodiments of the present disclosure, the preprocessor may process a sampling rate of the smart insole to 100Hz.

According to embodiments of the present disclosure, the preprocessor may further include a unit vectorizer configured to vectorize a unit format of each series of time series walking data obtained from the pressure sensor, the acceleration sensor, and the gyro sensors included in the smart insole, a unit minimum length vectorizer configured to find data having a minimum length in unit-format vectorized data for each of the pressure sensor, the acceleration sensor, and the gyro sensors, and equalize a length of the unit-format vectorized data to the minimum length, and a unit vector set part configured to construct a minimum unit format data set from minimum unit format data equally processed to the minimum length.

According to embodiments of the present disclosure, the unit vectorizer may perform a convolution operation using N pressure values and an average of a Gaussian function in order to vectorize the time series walking data in a unit format, and the convolution operation may be calculated according to

$$Z(t) = (X^p(t) * y)(t) = \int_0^t \overset{p}{X}(\tau) y(t-\tau) d\tau$$

(where $X^v(t)$ indicates an average of N pressure values,

$$y(t) = \frac{1}{\sqrt{2\pi}\sigma} e^{-\frac{t^2}{2\sigma^2}}$$

indicates the N pressure values, and $\sigma = 0.2_s$).

According to embodiments of the present disclosure, in a case where a sorted list for each foot is [t0,t1,....,ti.....],

$$\frac{d}{dx} z(t) = 0 \quad \text{and} \quad \frac{d^2}{dt^2} z(t) > 0$$

are applied with respect to every time t, and $t_i < t_{i+1}$, the unit vectorizer may define the time series walking data as a vectorized time series in a unit format $S_i(t) = x(t+t_i)$, and a discontinuous variable may be calculated according to $len(s_i(t)) = [(t_{i+1} - t_i) \times 100]$ because a sample speed of the insole is 100Hz and a standard length is defined as $d = \min_i len(s_i(t))$.

According to embodiments of the present disclosure, the ensemble learner may apply the ensemble learning model to a fully connected network and may include a CNN set constructor configured to construct a CNN series learning_vector_data set derived through the CNN series learning based on a minimum unit-format data set, and an RNN set constructor configured to construct an RNN series learning_vector_data set derived through the RNN series learning based on the minimum unit format data set.

According to embodiments of the present disclosure, the ensemble learner may further include a CNN-RNN set constructor configured to, in a test stage, construct an average data vector set of the CNN-series learning_vector_data set and the RNN-series learning_vector_data set to construct a final walking data set for identifying the user from the average data vector set. According to embodiments of the present disclosure, the CNN-based learning and the RNN-based learning may be respectively and independently trained using the CNN-series learning_vector_data set and the RNN-series learning_vector_data set, and, in a test stage, an individual's softmax scores may be calculated by taking an average of soft max scores in CNN and RNN

According to embodiments of the present disclosure, the CNN series learning and the RNN series learning may be defined as $M(s_i^p(t), s_i^\alpha(t), s_i^\gamma(t)) = u$ (for tri-modal sensing) where a unit step of $s_i^p(t)$ in a standard format, an acceleration $s_i^\alpha(t)$, and rotation $s_i^\gamma(t)$ is used as inputs an output of a model is a vector of a soft max probability u.

According to embodiments of the present disclosure, the CNN series learning and the RNN series learning may construct an average ensemble model to aggregating CNN and RNN predictions and provide one final prediction, and an average probability of CNN and RNN may be calculated according to $M_{ens} = \frac{1}{2}(M_{cnn} + M_{rnn})$ (where $M_{enn}$ indicates a case where only CNN is activated, $M_{rnn}$ indicates a case where only RNN is activated, and $M_{em}$ indicates a

case where CNN and RNN are all activated).

According to embodiments of the present disclosure, the ensemble learning model may be composed of vectors of 128 units in a dimension of embedding the CNN-series learning_vector_data set or the RNN-series learning_vector_data set, a CNN-based learning_vector and an RNN-based learning_vector may be connected to a fully connected network layer to form embedding vectors of 256 units, and the embedding vectors may be normalized to a same value.

According to embodiments of the present disclosure, the device may further include an output part configured to output, through the network trained with the ensemble learning model, walking feature data of the user from each unit format data set obtained from the sensors so as to identify (authenticate) the user from the walking data.

According to the multi-modal few-shot learning device for user identification using a walking pattern based on a deep learning ensemble as described above, the following effects are obtained.

First, by recognizing and excluding meaningless walking information data through a neural network trained with an ensemble learning model, it is possible to extract only feature data that enables identification of a user.

Second, by extracting the user's walking feature data without the meaningless information, it is possible to improve a probability of identifying the user.

Third, the learning effect is enhanced by using an ensemble model network that connects CNN and RNN networks.

Fourth, it is possible to extract feature walking data from walking data that enables identification of an individual through walking data in a situation where there is variation.

BRIEF DESCRIPTION OF THE DRAWINGS

FIG. 1 is a block diagram of the present disclosure.
FIG. 2 is a block diagram of a preprocessor according to an embodiment of the present disclosure.
FIGS. 3 and 4 are a block diagram and a schematic diagram of an ensemble learner, according to an embodiment of the present disclosure.
FIGS. 5 and 6 are a block diagram and a schematic diagram of a few-shot learner according to an embodiment of the present disclosure.
FIG. 7 is a graph showing a comparison of learning results of an ensemble model, a CNN model, and an RNN model according to an embodiment of the present disclosure.

DETAILED DESCRIPTION

A multi-modal few-shot learning device for user identification using a walking pattern based on deep learning ensemble according to embodiments of the present disclosure will be described in detail with reference to the accompanying drawings. The present disclosure may make various changes and have various forms, and specific embodiments will be illustrated in drawings and will be described in detail in the present specification. However, these are not intended to limit the present disclosure to specific disclosure forms, and it should be understood that the present disclosure includes all changes, equivalents, or substitutions falling within the spirit and scope of the present disclosure. In describing the drawings, like reference numerals are used for like elements. In the accompanying drawings, the dimensions of the structures might be shown exaggerated for clarity of the disclosure or abridged for a schematic repesentation of the configurations of some embodiments.

Also, the terms such as "first" and "second" may be used to describe various components, but those components should not be limited by the terms. The terms are merely used to distinguish one component from other components. For example, without departing from the scope of the present disclosure, a first component may be referred to as a second component, and similarly, a second component may also be referred to as a first component. Meanwhile, unless otherwise defined, all terms used herein, including technical or scientific terms, have the same meaning as commonly understood by those of ordinary skill in the art to which the present disclosure belongs. Unless otherwise defined, all terms, including technical and scientific terms, commonly used and defined in dictionaries are to be interpreted as is customary in the art to which the present disclosure belongs. It will be further understood that terms in common usage should also be interpreted as is customary in the related art and not in an idealized or overly formal sense unless expressly so defined herein.

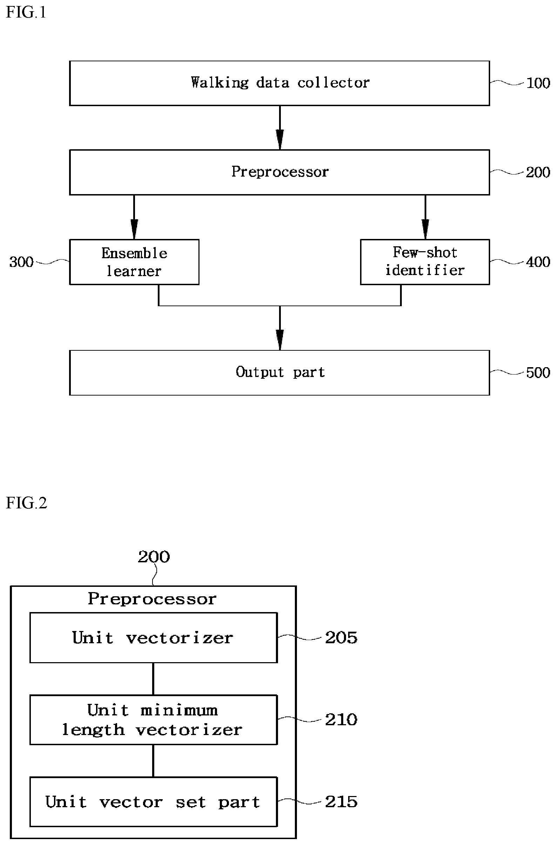

FIG. 1 is a block diagram of the present disclosure. Referring to FIG. 1, a multi-modal few-shot learning device for user identification using a walking pattern may include a walking data collector 100, a preprocessor 200, an ensemble learner 300, a few-shot identifier 400 and an output part 500.

In one embodiment of the present disclosure, the walking data collector 100 collects a user's walking data from a smart insole including any one or more of a pressure sensor, an acceleration sensor, and a gyro sensor, and collects the user's walking data measured by n pressure sensors, acceleration sensors, and gyro sensors included in the smart insole. In addition, in one embodiment of the present disclosure, the n pressure sensors measure a measurement level of foot pressure of both feet of the user as 0 or 1 or 2, and a sampling rate of the smart insole may be 100Hz.

In addition, in one embodiment of the present disclosure, the preprocessor 200 converts a series of time series walking data obtained from each of the sensors included in the smart insole into a unit data set, and the ensemble learner 300 applies an ensemble learning model that provides one final prediction by training CNN series learning and RNN series learning respectively and independently based on the unit-format data set generated by the preprocessor 200. In addition, the few-shot identifier 400 according to an embodiment of the present disclosure recognizes and excludes walking data having inadequate information (few-shot data) related to the user's walking data. Lastly, the output part 500 outputs a walking data feature for identifying the user, by using a value output from a fully connected network learned through the ensemble learner 300. That is, through the network which has learned the ensemble learning model for identifying (authenticating) the user from the walking data, the user's walking feature data is extracted from each of the unit data set obtained from the sensors. In more detail, in order to accurately extract feature data related to the user with a small amount of walking data related to the user, a function of the few-shot identifier 400 for discriminating whether the collected walking data is walking data related to the user or walking data related to a non-user and a function of the few-shot identifier 400 for learning the ensemble model in the network by inputting the walking data related to the user as an input may be performed in parallel.

FIG. 2 is a block diagram of a preprocessor according to an embodiment of the present disclosure.

Referring to FIG. 2, the preprocessor 200 may include a unit vectorizer 205, a unit minimum length vectorizer 210, and a unit vector set part 215.

In more detail, the unit vectorizer 205vectorizes a unit format of a series of time series walking data obtained from the pressure sensor, the acceleration sensor, and the gyro sensors included in the smart insole, and the unit minimum length vectorizer 210 finds data having a minimum length from among the unit-format vectorized data for each of the pressure sensor, the acceleration sensor, and the gyro sensor, and equalizes a length of the unit-format vectorized data to a minimum length. Next, the unit vector set part 215 may construct a minimum unit format data set according to the pressure sensor, the acceleration sensor, or the gyro sensor from a serioes of unit format data having the minimum length.

That is, in an optimal embodiment (best mode), the unit vectorizer 205 performs a convolution operation using N pressure values and an average of a Gaussian function to vectorize the time series walking data in a unit format. The

$$Z(t) = (X^p(t) *_y)(t) = \int_0^t \overset{p}{X}(\tau)_y(t-\tau)d\tau$$

convolution operation is calculated as (where $X^p(t)$ indicates an average of N pressure values, $y(t) = \frac{1}{\sqrt{2\pi\sigma}} e^{-\frac{t^2}{2\sigma^2}}$ indicates N pressure values, and $\sigma = 0.2_s$). In addition, in a case where a sorted list for each foot is [t0,t1,....,ti.....], $\frac{d}{dx}z(t) = 0$ and $\frac{d^2}{dt^2}z(t) > 0$ are applied with respect to every time t, and $t_i < t_{i+1}$, the time series walking data is defined as a vectorized time series in a unit format $S_i(t) = x(t+t_i)$ (where $0 \leq t \leq t_{i+1} - t_i$). Since a sampling rate of the insole is 100Hz and a standard length is defined as $d = min_i len(s_i(t))$, a discontinuous variable is calculated according to $len(s_i(t)) = [(t_{i+1} - t_i) \times 100]$ (where $S_i(t)$ indicates a unit step of two feet of every participant).

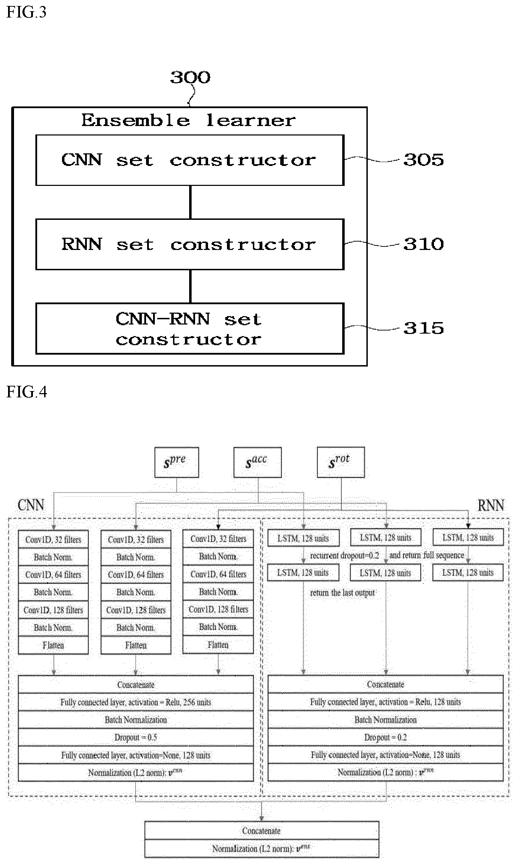

FIGS. 3 and 4 are a block diagram and a schematic diagram of an ensemble learner, according to an embodiment of the present disclosure. Referring to FIGS. 3 and 4, the ensemble learner 300 may include a CNN set constructor 305, an RNN set constructor 310, and a CNN-RNN set constructor 315.

In more detail, in order to accurately extract a user's walking feature data, big data related to the user's walking data is required. However, there is a limitation in learning CNN or RNN by collecting in advance the walking data according to the user's environmental condition. Therefore, through CNN learning and RNN learning only with a small amount of the walking data related to the user, the network is learned by applying an ensemble model, so that feature data of walking data related to the user can be derived for each environmental condition with various changes.

In more detail, according to an embodiment of the present disclosure, the ensemble learner 300 applies and learns an ensemble learning model to a fully connected network, and the CNN set constructor 305 constructs a CNN series learning_vector_data set derived through the CNN series learning based on a minimum unit format data set generated by the preprocess 200. The RNN set constructor 310 constructs an RNN series learning_vector_data set derived through the RNN series learning based on the minimum unit format data set. In addition, the CNN-RNN set constructor 310 constructs an average data vector set of the CNN-series learning_vector_data set and the RNN-series learning_vector_data set in a test stage to construct a final walking data set for identifying the user from the average data vector set.

In an optimal embodiment (best mode) of the present disclosure, the CNN-series learning and RNN-series learning are respectively and independently trained using the CNN-series learning_vector_data set and the RNN-series learning_vector_data set, and, in a test stage, an individual's softmax scores are calculated by taking an average of the softmax scores in CNN and RNN That is, the CNN series learning or the RNN series learning is defined as $M\left(s_i^p(t), s_i^o(t), s_i^{\gamma}(t)\right) = u$ (for tri-modal sensing) where an input is a unit step of $s_i^p(t)$ in a standard format, an acceleration $s_i^o(t)$, and rotation $s_i^{\gamma}(t)$ and an output of a model is a soft max probability u. In addition, the CNN series learning and the RNN series learning aggregate CNN and RNN predictions, construct an average ensemble model to provide one final prediction, and calculate an average probability of CNN and RNN according to $M_{ens} = \frac{1}{2}\left(M_{cnn} + M_{rnn}\right)$ (where $M_{cnn}$ indicates a case where only CNN is activated, $M_{rnn}$ indicates a case where only RNN is activated, and $M_{ern}$ indicates a case where CNN and RNN are all activated). In addition, the ensemble learning model is composed of vectors of 128 units in a dimension of embedding the CNN series learning_vector_data set or the RNN series learning_vector_data set, and a CNN-series learning_vector or an RNN-series learning_vector is connected to a fully connected network layer to form embedding vectors of 256 units, and the embedding vectors are normalized to the same value.

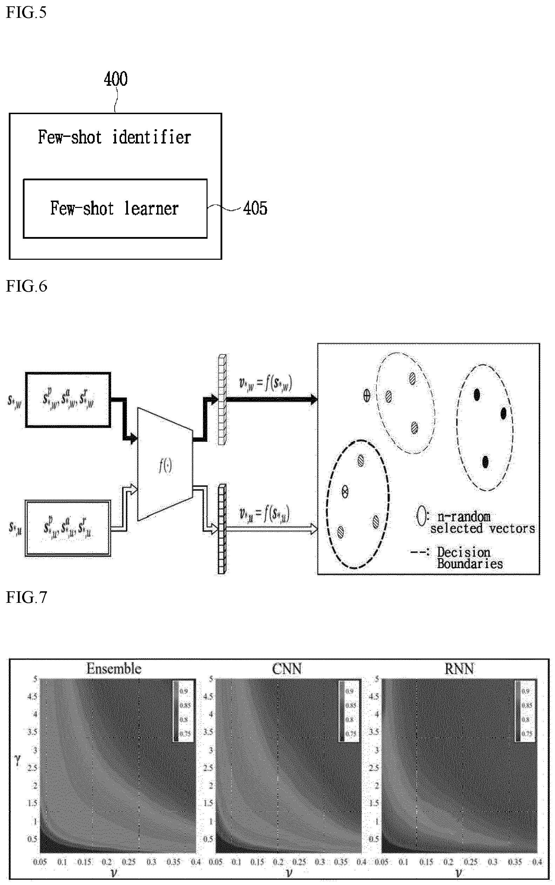

FIGS. 5 and 6 are a block diagram and a schematic diagram of a few-shot learner according to an embodiment of the present disclosure. Referring to FIGS. 5 and 6, the few-shot identifier 400 includes a few-shot learner 405.

In embodiments of the present disclosure, the few-shot identifier 400 includes a few-shot learner that utilizes a Support Vector Machine (SVM) to exclude walking data irrelevant to the user's walking data based on an inadequate information (few-shot data) set related to the user's walking data.

Here, the few-shot learner 405 includes the inadequate information (few-shot data) set related to the user's walking data, and the inadequate information (few-shot data) set related to the user's walking data includes data which is not walking data (unknown known data) and non-user data (unknown unknown data) in user-related data. Therefore, the few-shot learner 400 utilizes the SVM to exclude inadequate information (few-shot data) sets related to the user's walking data and the non-user data (unknown unknown data).

In an optimal embodiment of the few-shot learner 405, the few-shot learner sets a vector set $s_{i,\alpha}|1 \leq i \leq n$ in a unit format randomly selected from the non-user data (unknown unknown data) (where $3 \leq n \leq 10$) and calculates a center of n embedding vectors from an embedding vector set $\{V_{i,\alpha} = f(S_{i,\alpha})|1 \leq i \leq n\}$ generated by any one model of CNN and RNN networks (where the center of the embedding vector is $M_{\alpha} = \frac{1}{n}\sum_{i=1}^{n} V_{i,\alpha}$). The center of the embedding vectors and $\{V_{i,\alpha}|1 \leq i \leq n\}$ are input conditions, and, in order to solve the optimization, $\min_{\alpha} = \frac{1}{2}\sum_{i}^{n}\sum_{i}^{n}, \alpha_i \alpha_i$ and K($V_{i,\alpha}$, $V_{i,\alpha}$) are applied to the SVM (where $0 \leq \alpha_i \leq \frac{1}{vn}$, $\sum_{i=1}^{n} \alpha_i = 1$, $K(V, V') = e^{-\gamma\|v - v'\|_2^2}$ indicates a radial bias kernel function, $\alpha_i$ indicates a Lagrange multiplier, and $\gamma$ and V indicate hyperparameters).

FIG. 7 is a graph showing a comparison of learning results of an ensemble model, a CNN model, and an RNN model according to an embodiment of the present disclosure. Referring to FIG. 7, a distribution of ACC as a function of y and V for CNN, RNN, and ensemble models is shown in FIG. 7. Selection of y and V is an important condition for the overall recognition accuracy. FIG. 7 according to an optimal embodiment of the present disclosure shows a comparison of regions (light green to yellow regions) that accounts for 90% or more in area and shows that an area of the ensemble model is wider than areas of the CNN and RNN This indicates that the ensemble model has a weak dependence when selecting y and V, which affects robustness of a recognition result.

In embodiments of the present disclosure, a method for the multi-modal few-shot learning device for user identification using a walking pattern based on a deep learning ensemble includes collecting a user's walking data from a smart insole including any one or more of a pressure sensor, an acceleration sensor, and a gyro sensor in operation, converting a series of time series walking data obtained from each of the sensors included in the smart insole into each unit format data set in operation, training a network based on an ensemble learning model for extracting the user's features based on the unit format data set in operation, recognizing and excluding walking data having information irrelevant to the user in operation, and extracting user feature walking data to identify the user from the walking data through the network trained with the ensemble learning model in operation.

According to the multi-modal few-shot learning device for user identification using a walking pattern based on deep learning ensemble as described above, the following effects are obtained. First, by recognizing and excluding meaningless walking information data through a neural network trained with an ensemble learning model, it is possible to extract only feature data that enables identification of a user. Second, by extracting the user's walking feature data without the meaningless information, it is possible to improve a probability of identifying the user. Third, the learning effect is enhanced by using an ensemble model network that connects CNN and RNN networks. Fourth, it is possible to extract feature walking data from walking data that enables identification of an individual through walking data in a situation where there is variation.

Although the preferred embodiments of the present disclosure have been disclosed for illustrative purposes, those skilled in the art will appreciate that various modifications, additions and substitutions are possible, without departing from the scope and spirit of the invention as disclosed in the accompanying claims. Therefore, the above descriptions and the attached drawings should be interpreted as exemplifying the present disclosure rather than limiting the spirit of the present disclosure.

[Detailed Description of Main Elements]

| | |
|---|---|
| 100: walking data collector | 200: preprocessor |
| 205: unit vectorizer | 210: unit minimum length vectorizer |
| 215: unit vector set part | 300: Ensemble learner |
| 305: CNN set constructor | 310: RNN set constructor |
| 315: CNN-RNN set constructor | 400: few-shot identifier |
| 405: few-shot learner | 500: output part |

**Claims**

1. A multi-modal few-shot learning device for user identification using a walking pattern based on deep learning ensemble, the device comprising:

   a walking data collector 100 configured to collect walking data of a user from a smart insole including any one or more of a pressure sensor, an acceleration sensor, and a gyro sensor;
   a preprocessor 200 configured to convert a series of time series walking data obtained from each of the sensors included in the smart insole into a unit format data set; and
   an ensemble learner 300 configured to apply an ensemble learning model that provides one final prediction by training CNN series learning and RNN series learning respectively and independently based on the unit-format data set generated by the preprocessor 200.

2. The device of claim 1,
   wherein the walking data collector 100 comprises any one or more of n pressure sensors, the acceleration sensor, and the gyro sensor included in the smart insole, and collects the walking data of the user measured from the sensors.

3. The device of claim 2,
   wherein the n pressure sensors each measure a measurement level of foot pressure of both feet of the user is as 0, 1, or 2.

4. The device of claim 1,
   wherein the preprocessor 200 processes a sampling rate of the smart insole to 100Hz.

5. The device of claim 1,
   wherein the preprocessor 200 further comprises:

   a unit vectorizer 205 configured to vectorize a unit format of each series of time series walking data obtained from the pressure sensor, the acceleration sensor, and the gyro sensors included in the smart insole;
   a unit minimum length vectorizer 210 configured to find data having a minimum length in unit-format vectorized data for each of the pressure sensor, the acceleration sensor, and the gyro sensors, and equalize a length of the unit-format vectorized data to the minimum length; and

a unit vector set part 215 configured to construct a minimum unit format data set from minimum unit format data equally processed to the minimum length.

**6.** The device of claim 5,

wherein the unit vectorizer 205 performs a convolution operation using N pressure values and an average of a Gaussian function in order to vectorize the time series walking data in a unit format,

wherein the convolution operation is calculated according to $Z(t) = (X^p(t)*y)(t) = \int_0^t \overset{p}{X}(\tau)y(t-\tau)d\tau$

$$y(t) = \frac{1}{\sqrt{2\pi}\sigma} e^{-\frac{t^2}{2\sigma^2}}$$

(where $X^p(t)$ indicates an average of N pressure values, $\frac{1}{\sqrt{2\pi}\sigma} e^{-\frac{t^2}{2\sigma^2}}$ indicates the N pressure values, and $\sigma=0.2_s$).

**7.** The device of claim 5,

wherein, in a case where a sorted list for each foot is [t0, t1, ...., ti...], $\frac{d}{dx}z(t) = 0$ and $\frac{d^2}{dt^2}z(t) > 0$ are applied with respect to every time t, and $t_i < t_{i+1}$, the unit vectorizer defines the time series walking data as a vectorized time series in a unit format $S_i(t)=x(t+t_i)$,

wherein a discontinuous variable is calculated according to $len(s_i(t)) = [(t_{i+1} - t_i)\times100]$ because a sample speed of the insole is 100Hz and a standard length is defined as $d=\min_i len(s_i(t))$.

**8.** The device of claim 1,

wherein the ensemble learner 300 applies the ensemble learning model to a fully connected network and comprises:

a CNN set constructor 305 configured to construct a CNN series learning_vector_data set derived through the CNN series learning based on a minimum unit-format data set; and
an RNN set constructor 310 configured to construct an RNN series learning_vector_data set derived through the RNN series learning based on the minimum unit format data set.

**9.** The device of claim 8,

wherein the ensemble learner 300 further comprises a CNN-RNN set constructor 315 configured to, in a test stage, construct an average data vector set of the CNN-series learning_vector_data set and the RNN-series learning_vector_data set to construct a final walking data set for identifying the user from the average data vector set

**10.** The device of claim 8,

wherein the CNN series learning and the RNN series learning are respectively and independently trained using the CNN-series learning_vector_data set and the RNN-series learning_vector_data set, and
wherein, in a test stage, an individual's softmax scores are calculated by taking an average of soft max scores in CNN and RNN

**11.** The device of claim 8,

wherein the CNN series learning or the RNN series learning is defined as $M(s_i^p(t), s_i^\rho(t), s_i^\gamma(t)) = u$ (for tri-modal sensing) where a unit step of $s_i^p(t)$ in a standard format, an acceleration $s_i^\rho(t)$, and rotation $s_i^\gamma(t)$ is used as inputs an output of a model is a vector of a soft max probability u.

**12.** The device of claim 8,

wherein the CNN series learning and the RNN series learning construct an average ensemble model to aggregating CNN and RNN predictions and provide one final prediction, and an average probability of CNN and RNN is calculated according to $M_{ens} = \frac{1}{2}(M_{cnn} + M_{rnn})$ (where $M_{enn}$ indicates a case where only CNN is activated, $M_{rnn}$ indicates a case where only RNN is activated, and $M_{ern}$ indicates a case where CNN and RNN are all activated).

13. The device of claim 8,

wherein the ensemble learning model is composed of vectors of 128 units in a dimension of embedding the CNN-series learning_vector_data set or the RNN-series learning_vector_data set,
wherein a CNN-series learning_vector or an RNN-series learning_vector is connected to a fully connected network layer to form embedding vectors of 256 units, and
wherein the embedding vectors are normalized to a same value.

14. The device of claim 1, further comprising:
an output part 500 configured to output, through the network trained with the ensemble learning model, walking feature data of the user from each unit format data set obtained from the sensors so as to identify (authenticate) the user from the walking data.

FIG.1

| Walking data collector | ~100 |

↓

| Preprocessor | ~200 |

↓                                    ↓

300~ | Ensemble learner |          | Few-shot identifier | ~400

↓

| Output part | ~500 |

FIG.2

200

| Preprocessor |
| Unit vectorizer | ~205 |
| Unit minimum length vectorizer | ~210 |
| Unit vector set part | ~215 |

FIG.3

300

Ensemble learner

| CNN set constructor | 305 |

| RNN set constructor | 310 |

| CNN-RNN set constructor | 315 |

FIG.4

$s^{pre}$  $s^{acc}$  $s^{rot}$

CNN                                                                RNN

| Conv1D, 32 filters | Conv1D, 32 filters | Conv1D, 32 filters |
| Batch Norm. | Batch Norm. | Batch Norm. |
| Conv1D, 64 filters | Conv1D, 64 filters | Conv1D, 64 filters |
| Batch Norm. | Batch Norm. | Batch Norm. |
| Conv1D, 128 filters | Conv1D, 128 filters | Conv1D, 128 filters |
| Batch Norm. | Batch Norm. | Batch Norm. |
| Flatten | Flatten | Flatten |

| LSTM, 128 units | LSTM, 128 units | LSTM, 128 units |

recurrent dropout=0.2 and return full sequence

| LSTM, 128 units | LSTM, 128 units | LSTM, 128 units |

return the last output

Concatenate
Fully connected layer, activation = Relu, 256 units
Batch Normalization
Dropout = 0.5
Fully connected layer, activation=None, 128 units
Normalization (L2 norm): $v^{cnn}$

Concatenate
Fully connected layer, activation = Relu, 128 units
Batch Normalization
Dropout = 0.2
Fully connected layer, activation=None, 128 units
Normalization (L2 norm): $v^{rnn}$

Concatenate
Normalization (L2 norm): $v^{ens}$

FIG.5

400

Few-shot identifier

Few-shot learner — 405

FIG.6

$s^*_w$   $s^p_{*w}, s^a_{*w}, s^r_{*w}$   $v^*_w = f(s^*_w)$

$s^*_u$   $s^p_{*u}, s^a_{*u}, s^r_{*u}$   $v^*_u = f(s^*_u)$

$f(\cdot)$

◯: n-random selected vectors

--: Decision Boundaries

FIG.7

Ensemble    CNN    RNN

$\gamma$

$v$    $v$    $v$

Europäisches
Patentamt

European
Patent Office

Office européen
des brevets

# EUROPEAN SEARCH REPORT

Application Number

EP 21 19 4284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOON JUCHEOL ET AL: "Multimodal Few-Shot Learning for Gait Recognition", APPLIED SCIENCES, vol. 10, no. 21, 29 October 2020 (2020-10-29), pages 1-15, XP55890949, DOI: 10.3390/app10217619 | 1-5,8, 11,13,14 | INV. G06K9/62 G06N3/04 G06V10/82 G06V40/20 A61B5/11 A61B5/00 |
| Y | * abstract * <br> * Section 1. * <br> * Sections 2.1, 2.2, 2.3, 2.4, 2.5, 2.7 * <br> * Section 3.1 * <br> * figures 1, 2 * | 6,7,9, 10,12 | |
| Y | MOON JUCHEOL ET AL: "Can Ensemble Deep Learning Identify People by Their Gait Using Data Collected from Multi-Modal Sensors in Their Insole?", SENSORS, vol. 20, no. 14, 18 July 2020 (2020-07-18) , pages 1-15, XP055890973, DOI: 10.3390/s20144001 <br> * Sections 3., 3.4 * <br> * Sections 2.2.1, 2.2.2 * <br> * figure 5 * | 6,7,9, 10,12 | |

-----

-----

TECHNICAL FIELDS
SEARCHED      (IPC)

G06K
G06N
G06V
A61B

The present search report has been drawn up for all claims

| Place of search | Date of completion of the search | Examiner |
|---|---|---|
| **The Hague** | **15 February 2022** | **Moreno, Marta** |

EPO FORM 1503 03.82 (P04C01)

**EP 4 113 374 A1**

**Patent documents cited in the description**

- KR 102175191
- KR 102061810
- KR 102194313